# EUROPÄISCHE PATENTSCHRIFT

(11) **EP 3 542 761 B1**
(45) Veröffentlichungstag und Bekanntmachung des Hinweises auf die Patenterteilung: **25.11.2020**
(21) Anmeldenummer: 19158716.1
(22) Anmeldetag: 22.02.2019
(51) Int. Cl.: A61F 2/38

(54) **TIBIA-KOMPONENTE EINES KÜNSTLICHEN KNIEGELENKS**
TIBIA COMPONENT OF AN ARTIFICIAL KNEE JOINT
COMPOSANT TIBIA D'UNE ARTICULATION ARTIFICIELLE DU GENOU

(30) Priorität: 20.03.2018 DE 102018106541
(43) Veröffentlichungstag der Anmeldung: 25.09.2019
(73) Patentinhaber: ARTIQO GmbH, 59348 Lüdinghausen (DE)
(72) Erfinder: Bücken, Ulrich, 59348 Lüdinghausen (DE); Frank, Thomas Mario, 7000 Eisenstadt (AT)
(74) Vertreter: Gosdin, Michael

(56) Entgegenhaltungen:
- EP-A1- 3 231 396
- DE-A1-102014 106 999
- DE-A1-102016 114 059
- DE-T2- 69 529 200

## Beschreibung

Die Erfindung betrifft eine Tibia-Komponente eines künstlichen Kniegelenks, umfassend eine Auflageplatte an der ein Verankerungskiel zur Verankerung in der Tibia des Trägers des Kniegelenks angeordnet ist, wobei der Verankerungskiel einen ersten Wandungsabschnitt und einen zweiten Wandungsabschnitt aufweist, die miteinander verbunden sind, wobei beide Wandungsabschnitte in einer Ansicht anterior-posterior zumindest über einen vorgegebenen Umfangsabschnitt eine konvex geformte Außenform aufweisen.

Eine solche Tibia-Komponente ist aus der DE 10 2016 114 059 A1 bekannt. Eine ähnliche Lösung zeigt die DE 695 29 200 T2**.** In der DE 10 2014 106 999 A1 ist ein Femurimplantat als Teil einer Knieprothese offenbart, welches eine weitere ähnliche Tibia-Komponente umfasst. Auf einer Auflageplatte ist der Verankerungskiel angeordnet, der bei der Implantation ins Innere der Tibia (d. h. des Schienbeins) eingebracht wird. Der gemäß dieser Lösung vorbekannte Kiel ist dabei im wesentlichen stabförmig (turmartig) ausgebildet und hat insgesamt eine längliche Erstreckung mit einem endseitig angeordneten Schaft. Näher an der Auflageplatte angeordnet ist ein flügelartig ausgebildetes Verankerungselement.

Bei der vorbekannten Lösung besteht das Problem, dass mitunter in nachteiliger Weise bei der Implantation die Kompakta des Tibia-Knochens beeinträchtigt wird, nämlich dann, wenn die Tibia-Komponente in mediallateraler Richtung, den anatomischen Gegebenheiten des Patienten folgend, schräg nach lateral geneigt eingebaut wird. Ist diesen Fällen kann das Ende des genannten Schafts im Inneren des Markraums die Kompakta des Tibia-Knochens touchieren, das sogenannte Impingement-Phänomen, das beim Patienten Schmerzen verursacht.

Auch wirkt das anterior-posteriore Röntgenbild des Kniegelenks bei solchermaßen versorgten Patienten optisch beunruhigend.

Der vorliegenden Erfindung liegt die **Aufgabe** zugrunde, eine Tibia-Komponente der eingangs genannten Art bereitzustellen, mit der es möglich ist, bei der Implantation der Komponente in die Tibia die Kompakta des Knochens möglichst wenig zu beeinflussen, womit ein verbessertes Operationsergebnis erreicht werden soll, und gleichzeitig eine "optisch verzeihende" Darstellung im Röntgenbild zu erreichen. Dennoch soll ein guter Sitz der Komponente im bzw. auf dem Knochen sichergestellt sein. Weiterhin wird angestrebt, eine verbesserte Lastübertragung und homogenere Knochenbelastung durch das Implantat zu erreichen.

Die **Lösung** dieser Aufgabe durch die Erfindung ist dadurch gekennzeichnet, dass die beiden Wandungsabschnitte in der Ansicht anterior-posterior eine im wesentlichen halbkreisförmige oder halb-elliptische Gestalt aufweisen, wobei der Verankerungskiel hinsichtlich der Richtung lateral-medial sowie hinsichtlich der Richtung anterior-posterior zentriert zum Mittelpunkt der Auflageplatte auf der Auflageplatte angeordnet ist.

Die beiden Wandungsabschnitte sind in einer Draufsicht auf die Auflageplatte bevorzugt in einem Winkel zueinander angeordnet, der zwischen 90° und 180° beträgt, besonders bevorzugt zwischen 110° und 150°.

Die beiden Wandungsabschnitte können alternativ in einer Draufsicht auf die Auflageplatte auch die Form eines Kreisbogens, eines Teils einer Ellipse, einer Parabel oder eines Vielecks aufweisen.

Der Verankerungskiel weist nach einer Fortbildung einen dritten Wandungsabschnitt auf. Dieser dritte Wandungsabschnitt wird auch als Finne bezeichnet. Er stellt eine Erhöhung der Rotationsstabilität der Tibia-Komponente sicher.

Die Finne kann in der Draufsicht auf die Auflageplatte im wesentlichen winkelhalbierend zwischen dem ersten und zweiten Wandungsabschnitt angeordnet sein. Weiterhin sieht eine Fortbildung vor, dass die Finne in einer Ansicht medial-lateral zumindest über einen vorgegebenen Umfangsabschnitt eine konkav geformte Außenform, eine konvex geformte Außenform oder eine gerade verlaufende oder abgeschrägte Außenform aufweist.

Der Übergangsbereich zwischen dem ersten Wandungsabschnitt und dem dritten Wandungsabschnitt und zwischen dem zweiten Wandungsabschnitt und dem dritten Wandungsabschnitt ist nach einer Fortbildung konkav ausgestaltet.

Die Dicke der Wandungsabschnitte beträgt bevorzugt zwischen 1 und 8 mm, besonders bevorzugt zwischen 2 und 4 mm. Dabei kann vorgesehen sein, dass die Dicke der Wandungsabschnitte über die Erstreckung der Wandung im wesentlichen konstant bleibt. Allerdings kann auch vorgesehen werden, dass die Dicke der Wandungsabschnitte in Richtung medial-lateral (für den ersten bzw. zweiten Wandungsabschnitt) bzw. in Richtung anterior-posterior (für den dritten Wandungsabschnitt) abnimmt. In ähnlicher Weise kann für alle drei Wandungsabschnitte vorgesehen werden, dass diese in Richtung proximal-distal dünner werden.

Der erste und/oder der zweite Wandungsabschnitt können zumindest abschnittsweise zur Richtung normal auf die Auflageplatte unter einem Winkel angeordnet sein, der zwischen 0° und 7° beträgt. Demgemäß ist eine Verkippung der Auflageplatte relativ zur Vertikalachse des Kiels (nach dorsal) vorgesehen (sog."Slope").

Die Auflageplatte kann eine Aufnahme für ein Inlay umfassen, wobei das Inlay insbesondere durch eine formschlüssige Verbindung, besonders bevorzugt durch eine Schnappverbindung, mit der Aufnahme verbindbar ist. Auch eine Schraubverbindung ist möglich. Das Inlay (welches als solches bekannt ist; das Material ist häufig UHMWPE (Ultra-high-molecular-weight polyethylene) oder hochvernetztes UHMEPE, mit oder ohne Vitamin E) stellt quasi einen Ersatz für den Meniskus dar und ist mit der Aufnahme verbindbar.

Die Schnappverbindung kann durch einen simsartigen Rand an der Aufnahme realisiert sein, in den das Inlay hineingedrückt wird und dabei einschnappt. Wie gesagt, kann auch alternativ eine Schraubverbindung für die Aufnahme des Inlays an der Aufnahmeplatte vorgesehen werden oder im Falle eines Rotationsinlays eine zentrale Zapfenführung.

Im Regelfall werden die Auflageplatte und der Verankerungskiel als einstückiges Bauteil ausgeführt. Vorgesehen werden kann allerdings auch, dass die Auflageplatte und der Verankerungskiel als separate Bauteile hergestellt und miteinander verbunden sind. Der Verankerungskiel kann dabei mit verschiedenen Winkeln bezüglich seiner Grundlinie bzw. Grundfläche hergestellt werden, über die er mit der Auflageplatte in Verbindung steht. Damit kann individuellen Wünschen an die Ausgestaltung der Tibia-Komponente Rechnung getragen werden.

Die Richtung anterior-posterior (a-p-Richtung) bedeutet von vorne nach hinten bei aufrecht stehender Person, die die in Rede stehende Tibia-Komponente trägt. Diese Richtung steht im wesentlichen senkrecht auf einer Normalen auf der Auflageplatte.

Die Richtung medial-lateral (m-1-Richtung) bedeutet von der Mitte nach seitlich bei aufrecht stehender Person, die die in Rede stehende Tibia-Komponente trägt. Auch diese Richtung steht im wesentlichen senkrecht auf einer Normalen auf der Auflageplatte.

Die Richtung proximal-distal bedeutet von nahe der Körpermitte zu fern der Körpermitte. Diese Richtung steht im wesentlichen senkrecht auf der Auflageplatte.

Die Positionierung des Kiels auf der Auflageplatte kann in verschiedener Weise erfolgen. Die erfindungsgemäße Ausführungsform sieht vor, dass der Kiel hinsichtlich der Richtung lateral-medial symmetrisch auf der Auflageplatte angeordnet ist, d. h. zentriert zum Mittelpunkt der Auflageplatte.

In ähnlicher Weise ist erfindungsgemäß der Kiel auf der Auflageplatte in Richtung anterior-posterior symmetrisch auf derselben angeordnet, d. h. zentriert zum Mittelpunkt der Auflageplatte.

Die Verankerung der Aufnahmeplatte in der Tibia kann unzementiert oder zementiert erfolgen.

Im Bereich des Zenits des Verankerungskiels (d. h. an dem von der Auflageplatte abgewandten Ende desselben) kann ein stabartiger Schaft angeformt bzw. angeordnet sein, der zum entsprechenden Eindringen ins Innere der Tibia ausgebildet ist. Diese Ausgestaltung bietet die Möglichkeit, den anatomischen Gegebenheiten des individuellen Patienten Rechnung zu tragen, indem der Schaft unter verschiedenen Winkeln relativ zur senkrechten Achse der Tibia-Komponente angeordnet wird. Dabei kann eine Neigung sowohl in anterior-posterior-Richtung als auch in medial-lateral-Richtung vorgesehen sein, sowie auch eine Kombination derselben.

Die vorgeschlagene Tibia-Komponente kann als Sortiment mit ansteigender Größe bereitgestellt und eingesetzt werden. So sind beispielsweise zehn verschiedene Größen im Sortiment möglich, um möglichst viele Patientengruppen abzudecken. Die Komponenten können auch in Gruppen bzw. Spektren zusammengefasst werden.

Als Material für die Tibia-Komponente hat sich insbesondere eine Kobalt-Chromlegierung bewährt, sowie eine Titanlegierung oder Edelstahl.

Es hat sich gezeigt, dass durch die Kombination der oben genannten Merkmale eine hinsichtlich der Druckverteilung im Knochen ideale bzw. optimale Situation selbst dann erzielt werden kann, wenn, wie zunehmend geübt, die Tibiaplatte im physiologischen 87°-Winkel zur Tibiaachse eingebaut wird. Bei dem vorbekannten Kiel-Design gemäß dem eingangs genannten Stand der Technik würden sich im Trabekelwerk des Knochens hingegen Imbalanzen mit Druckmaxima und Druckminima ergeben, die zu unerwünschten Knochenumbauvorgängen führen. Diese sind, wie man zwischenzeitlich weiß, für einige der postoperativen Beschwerden nach Implantation eines künstlichen Kniegelenks verantwortlich. In der Konsequenz ist die halbrunde/halb-elliptische und zentrierte Ausführung des Kiels von entscheidender Bedeutung für das postoperative Wohlergehen des Patienten und ein positives Langzeitergebnis dieses äußerst invasiven Eingriffs.

Hinsichtlich der Lastübertragung gilt folgendes: Bei einer anatomischen Positionierung fällt im Mittel das Tibiaplateau um 3° nach medial ab, gleichzeitig richtet sich der Kiel (bei einer herkömmlichen Standardprothese) um diese 3° nach lateral. Die Schräglage des Kiels hat zur Folge, dass die Lastübertragung und Knochenbelastung in der proximalen Tibia asymmetrisch verläuft und daher unphysiologische Knochenreaktionen auftreten können.

Dieser Effekt ist bei einer erfindungsgemäßen runden/ovalen Ausgestaltung des Tibiakiels deutlich geringer ausgeprägt, da die runde/ovale Form keine konische Achse aufweist und die anatomische Adaptierung der Prothese toleriert.

Die obige Bewertung ließ sich durch die Patentanmelderin über eine Finite-Element-Analyse bestätigen, die die Spannungsverteilung in einer erfindungsgemäßen Tibia-Komponente nach deren Implantation angibt. Ziel war die Ermittlung der spezifischen Spannungsverteilung innerhalb des proximalen Schienbeinknochens.

Getestet wurde das Implantat in zwei Ausrichtungen relativ zur Gelenkachse: Erstens bei einer horizontalen Implantation gemäß der bislang üblichen mechanischen Gelenkachse (senkrecht zur Beinachse = 90°). Zweitens bei einer 3° nach medial verkippten Implantation gemäß der nach neuen Erkenntnissen physiologischen Gelenkachse (3° zur Beinachse = 93°).

Diese beiden Implantationsarten wurden wiederum drei Szenarien unterworfen, nämlich einer normalen Implantation, einer um 5° axial nach innen verdrehten Implantation und einer um 5° axial nach außen verdrehten Implantation. Auf diese Weise wurden außer dem Normalfall auch zwei extreme Fehlausrichtungen des Implantats getestet.

Für die normale Implantation konnte nachgewiesen werden, dass über 90 % des Schnittvolumens in der physiologischen Belastungsverteilungszone liegen, also in einem Bereich, in welchem keine signifikanten Knochenveränderungen (Knochenabbau, Knochenverdichtungen) nach der Implantation zu erwarten sind.

Prinzipiell galt dies auch für die simulierten Fehlausrichtungen mit axialer Verdrehung um 5° nach außen oder 5° nach innen. Allerdings zeigte sich bei beiden Bedingungen eine deutliche Veränderung in der Belastungsverteilung entlang des Schienbeinknochens. Dies impliziert eine gewisse Empfindlichkeit gegenüber einer rotatorischen Fehlausrichtung. Gleichwohl ergab die Analyse, dass auch bei diesen Fehlausrichtungen keine signifikanten Knochenveränderungen nach der Implantation zu erwarten sind.

Das runde/ovale Design hat diesbezüglich geringere Unterschiede gezeigt als die bisher üblichen tibialen Komponenten und somit bewiesen, dass damit, im Gegensatz zum herkömmlichen Design, eine anatomische Justierbarkeit bei homogener Spannungsverteilung möglich ist.

Hieraus ergab sich der Schluss: Obwohl (bei 5° Malrotation nach außen oder innen) offensichtliche Veränderungen beobachtet wurden, deutet die Tatsache, dass sich unter allen analysierten Bedingungen nur etwa 2 % der Belastungsverteilung im proximalen Aspekt des Schienbeins in der überbeanspruchten Zone befinden, darauf hin, dass es offensichtlich keinen negativen Einfluss auf die Lastverteilung mit dem abgerundeten Implantat bei physiologischen oder horizontalen Implantationen gibt. Die Möglichkeit, das abgerundete Design mit einer physiologischen Schnittführung (3° lateral) zusammen mit einer patientenspezifischen Instrumentierung zu implantieren, könnte bei Varuspatienten ("O-Beine") wünschenswert sein, da dann eine operative Entspannung des lateralen Bandes, des hinteren Kreuzbandes und der retinakulären Bänder nicht erforderlich ist. Dies könnte zu weniger Operationstrauma und einer schnelleren Genesung führen, wie es auch eine korrekte Lastübertragung gewährleistet.

Damit ergibt sich, dass aus der vorgeschlagenen spezifischen Gestaltung der Tibia-Komponente wesentliche Vorteile resultieren, die mit den vorbekannten Lösungen gemäß dem Stand der Technik nicht erreichbar sind.

In der Zeichnung ist ein Ausführungsbeispiel der Erfindung dargestellt. Es zeigen:
- Fig. 1: in perspektivischer Darstellung eine Tibia-Komponente, die Bestandteil einer Knieprothese ist, wobei die distale Richtung nach oben und die proximale Richtung nach unten weist,
- Fig. 2: in perspektivischer Darstellung die Tibia-Komponente, betrachtet aus einer anderen Richtung,
- Fig. 3: die Seitenansicht der Tibia-Komponente, betrachtet in Richtung medial-lateral und
- Fig. 4: die Draufsicht auf die Tibia-Komponente von distal.

In den Figuren ist in verschiedenen Ansichten eine Tibia-Komponente 1 dargestellt, die bei der Implantation eines künstlichen Kniegelenks mit der Tibia verbunden wird. Hierzu weist die Komponente 1 eine Auflageplatte 2 auf, auf der ein Verankerungskiel 3 angeordnet ist. Der sich von der Oberfläche der Auflageplatte 2 weg erstreckende Verankerungskiel 3 weist somit in die Richtung distal d (d. h. bei aufrechtstehender Person, die die Komponente 1 trägt, nach unten); in den Figuren ist die entsprechende Lage gekennzeichnet, wobei die Richtung distal d, die Richtung proximal pr, die Richtung anterior a, die Richtung posterior p, die Richtung medial m sowie die Richtung lateral 1 angegeben ist.

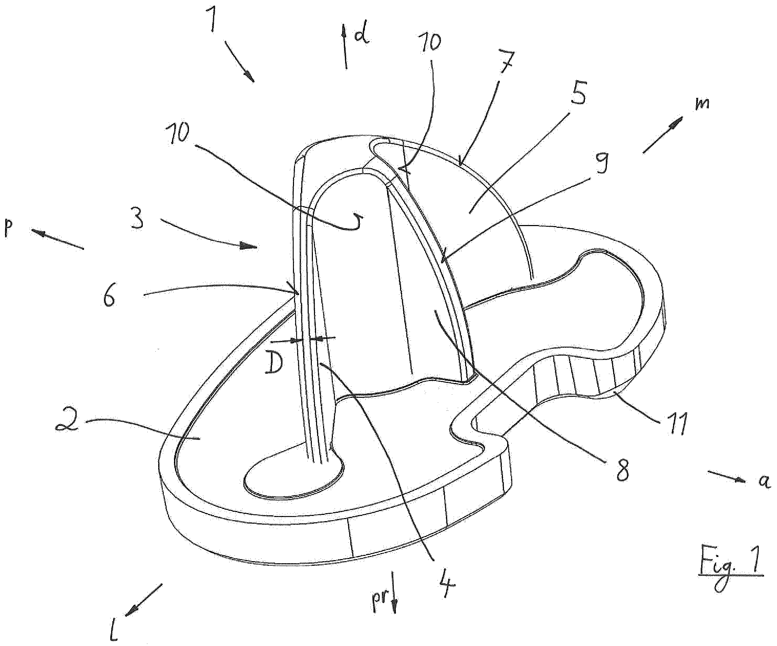

Wesentlich ist, dass der Verankerungskiel 3 einen ersten Wandungsabschnitt 4 und einen zweiten Wandungsabschnitt 5 aufweist, die miteinander verbunden sind; beide Wandungsabschnitte 4 und 5 weisen in einer Ansicht anterior-posterior a-p zumindest über einen vorgegebenen Umfangsabschnitt eine konvex geformte Außenform auf, die mit 6 bzw. mit 7 in den Figuren 1 bis 3 markiert ist.

Im Unterschied zu vorbekannten Lösungen, wo sich der Kiel von der Auflageplatte 2 im wesentlichen turmartig erstreckt, ist also eine im wesentlichen bogenförmige Gestaltung des Umrisse des Kiels 4 vorgesehen, wenn die Komponente 1 in Richtung anterior-posterior betrachtet wird.

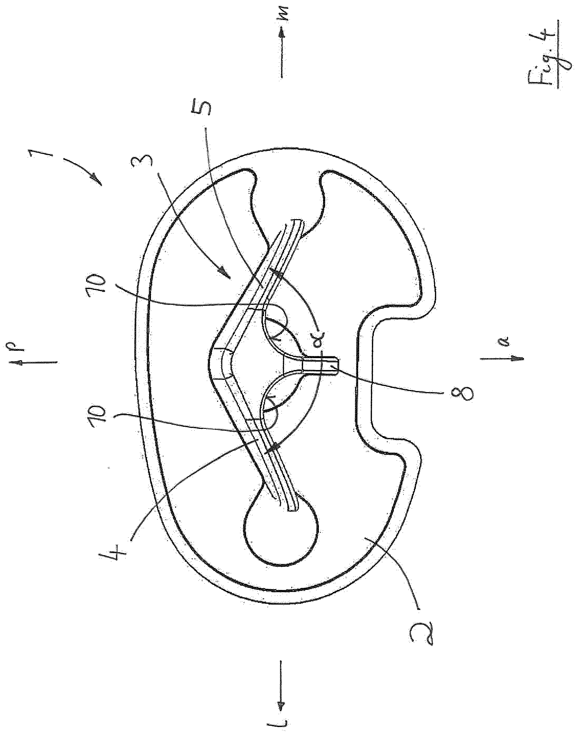

Quasi als eine Art Winkelhalbierende - am besten zu erkennen in Figur 4 - ist zwischen den beiden Wandungsabschnitten 4 und 5 ein dritter Wandungsabschnitt 8, d. h. eine Finne, angeordnet. Für die Außenform 9 des dritten Wandungsabschnitts 8 ist im Ausführungsbeispiel (siehe hierzu am besten Figur 1) eine konvexe Gestaltung vorgesehen, was allerdings nicht zwingend ist.

Wie aus Figur 4 zu erkennen ist, sind die beiden Wandungsabschnitte 4 und 5 in einem Winkel zueinander angeordnet, der mit α bezeichnet ist und der im Ausführungsbeispiel ca. 130 ° beträgt.

Der Übergangsbereich zwischen dem ersten Wandungsabschnitt 4 und dem dritten Wandungsabschnitt 8 sowie zwischen dem zweiten Wandungsabschnitt 5 und dem dritten Wandungsabschnitt 8 ist konkav ausgestaltet, s. konkaven Übergangsbereich 10, wie es sich am besten aus Figur 4 ergibt.

Die Dicke der Wandungsabschnitte ist in Figur 1 mit D markiert. Ein weitgehend konstanter Wert der Dicke ist möglich, allerdings auch eine Lösung, bei der die Dicke in Richtung medial m, lateral 1 bzw. anterior a allmählich abnimmt; gleichermaßen kann die Dicke in Richtung distal abnehmen.

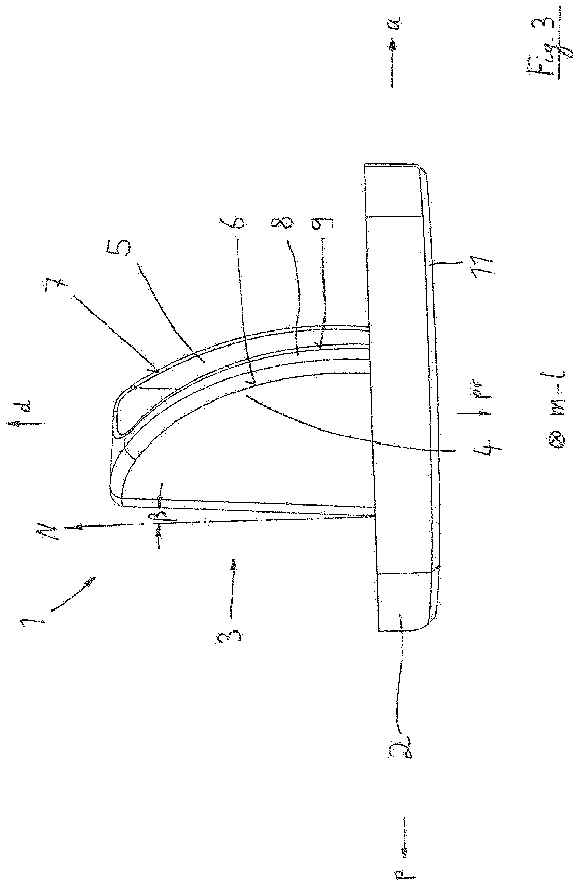

Die Ausrichtung der Wandungsabschnitte 4 und 5 auf der Auflageplatte 2 kann mit einem "Slope" versehen sein, wie es in Figur 3 illustriert ist. Hier ist die Normale N auf der Auflageplatte 2 eingetragen und der Winkel β, um den die Wandungsabschnitte geneigt sind.

Daraus ergibt sich bei eingebauter Tibia-Komponente in der anteriorposterioren Ansicht eine Neigung derselben nach posterior, wenn der Chirurg den Verankerungskiel in der Tibia streng proximal-distal ausrichtet.

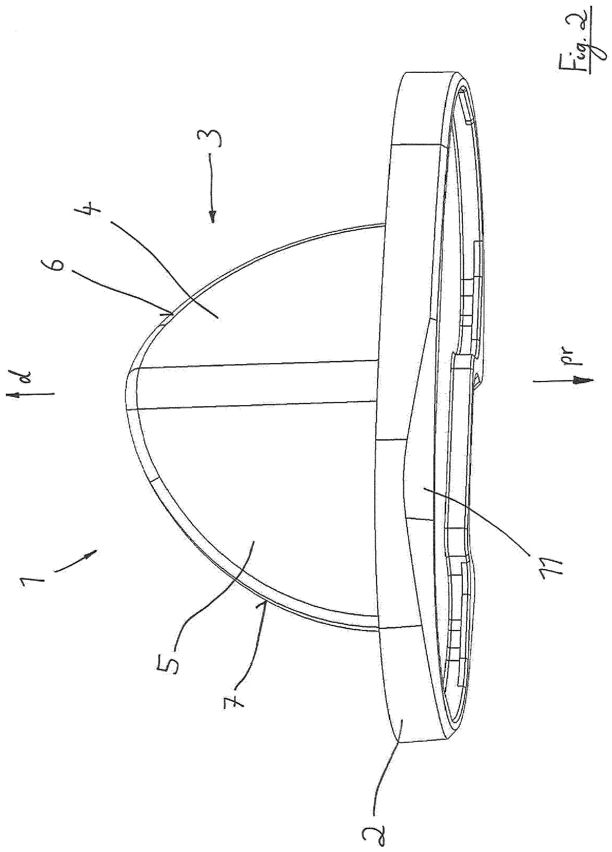

Am proximalen Rand der Auflageplatte ist eine Aufnahme 11 für ein Inlay (quasi als Ersatz für den Meniskus) vorgesehen (s. Figur 2). Das (selber nicht dargestellte) Inlay besteht bevorzugt aus Kunststoff (Polyäthylen). Die Aufnahme 11 fixiert das Inlay auf der Auflageplatte, bevorzugt nach Art einer Schnappverbindung.

### Bezugszeichenliste:

- 1: Tibia-Komponente
- 2: Auflageplatte
- 3: Verankerungskiel
- 4: erster Wandungsabschnitt
- 5: zweiter Wandungsabschnitt
- 6: Außenform des ersten Wandungsabschnitts
- 7: Außenform des zweiten Wandungsabschnitts
- 8: dritter Wandungsabschnitt (Finne)
- 9: Außenform des dritten Wandungsabschnitts
- 10: konkaver Übergangsbereich
- 11: Aufnahme für Inlay

- α: Winkel
- β: Winkel (Slope)
- D: Dicke des Wandungsabschnitts
- N: Normale auf der Auflageplatte

- a: anterior
- p: posterior
- m: medial
- l: lateral
- d: distal
- pr: proximal

## Patentansprüche

1. Tibia-Komponente (1) eines künstlichen Kniegelenks, umfassend eine Auflageplatte (2) an der ein Verankerungskiel (3) zur Verankerung in der Tibia des Trägers des Kniegelenks angeordnet ist, wobei der Verankerungskiel (3) einen ersten Wandungsabschnitt (4) und einen zweiten Wandungsabschnitt (5) aufweist, die miteinander verbunden sind, wobei beide Wandungsabschnitte (4, 5) in einer Ansicht anterior-posterior zumindest über einen vorgegebenen Umfangsabschnitt eine konvex geformte Außenform (6, 7) aufweisen,
**dadurch gekennzeichnet, dass**
die beiden Wandungsabschnitte (4, 5) in der Ansicht anterior-posterior eine im wesentlichen halbkreisförmige oder halb-elliptische Gestalt aufweisen, wobei der Verankerungskiel (3) hinsichtlich der Richtung lateral-medial sowie hinsichtlich der Richtung anterior-posterior zentriert zum Mittelpunkt der Auflageplatte (2) auf der Auflageplatte (2) angeordnet ist.

2. Tibia-Komponente nach Anspruch 1, **dadurch gekennzeichnet, dass** die beiden Wandungsabschnitte (4, 5) in einer Draufsicht auf die Auflageplatte (2) in einem Winkel (α) zueinander angeordnet sind, der zwischen 90° und 180° beträgt, vorzugsweise zwischen 110° und 150°.

3. Tibia-Komponente nach Anspruch 1, **dadurch gekennzeichnet, dass** die beiden Wandungsabschnitte (4, 5) in einer Draufsicht auf die Auflageplatte (2) die Form eines Kreisbogens, eines Teils einer Ellipse, einer Parabel oder eines Vielecks aufweisen.

4. Tibia-Komponente nach einem der Ansprüche 1 bis 3, **dadurch gekennzeichnet, dass** der Verankerungskiel (3) einen dritten Wandungsabschnitt (8) aufweist.

5. Tibia-Komponente nach Anspruch 4, **dadurch gekennzeichnet, dass** der dritte Wandungsabschnitt (8) in der Draufsicht auf die Auflageplatte (2) im wesentlichen winkelhalbierend zwischen dem ersten und zweiten Wandungsabschnitt (4, 5) angeordnet ist.

6. Tibia-Komponente nach Anspruch 4 oder 5, **dadurch gekennzeichnet, dass** der dritte Wandungsabschnitt (8) in einer Ansicht medial-lateral zumindest über einen vorgegebenen Umfangsabschnitt eine konkav geformte Außenform (9), eine konvex geformte Außenform (9) oder eine gerade verlaufende oder abgeschrägte Außenform (9) aufweist.

7. Tibia-Komponente nach einem der Ansprüche 5 oder 6, **dadurch gekennzeichnet, dass** der Übergangsbereich zwischen dem ersten Wandungsabschnitt (4) und dem dritten Wandungsabschnitt (8) und zwischen dem zweiten Wandungsabschnitt (5) und dem dritten Wandungsabschnitt (8) konkav (10) ausgestaltet ist.

8. Tibia-Komponente nach einem der Ansprüche 1 bis 7, **dadurch gekennzeichnet, dass** die Dicke (D) der Wandungsabschnitte (5, 6, 8) zwischen 1 und 8 mm beträgt, vorzugsweise zwischen 2 und 4 mm.

9. Tibia-Komponente nach einem der Ansprüche 1 bis 8, **dadurch gekennzeichnet, dass** der erste und/oder der zweite Wandungsabschnitt (5, 6) zumindest abschnittsweise zur Richtung normal (N) auf die Auflageplatte (2) unter einem Winkel (β) zwischen 0° und 7° angeordnet ist.

10. Tibia-Komponente nach einem der Ansprüche 1 bis 9, **dadurch gekennzeichnet, dass** die Auflageplatte (2) eine Aufnahme (11) für ein Inlay umfasst, wobei das Inlay insbesondere durch eine formschlüssige Verbindung, besonders bevorzugt durch eine Schnappverbindung, mit der Aufnahme (11) verbindbar ist.

11. Tibia-Komponente nach einem der Ansprüche 1 bis 10, **dadurch gekennzeichnet, dass** die Auflageplatte (2) und der Verankerungskiel (3) als separate Bauteile hergestellt und miteinander verbunden sind.

## Claims

1. Tibial component (1) of an artificial knee joint, comprising a support plate (2) on which an anchoring keel (3) for anchoring in the tibia of the wearer of the knee joint is arranged, wherein the anchoring keel (3) having a first wall section (4) and a second wall section (5) which are connected to one another, wherein both wall sections (4, 5) having a convex outer shape (6, 7) in an anterior-posterior view at least over a predetermined circumferential section,
**characterized in that**
the two wall sections (4, 5) have a substantially semicircular or semi-elliptical shape in the anterior-posterior view, wherein the anchoring keel (3) being arranged on the support plate (2) centered on the support plate (2) with respect to the lateral-medial direction and centered with respect to the anterior-posterior direction relative to the center of the support plate (2).

2. Tibial component according to claim 1, **characterized in that** the two wall sections (4, 5) are arranged at an angle (α) to one another in a plan view of the support plate (2), which is between 90° and 180°, preferably between 110° and 150°.

3. Tibial component according to claim 1, **characterized in that** the two wall sections (4, 5) have the shape of a circular arc, of a part of an ellipse, of a parabola or of a polygon in a plan view of the support plate (2).

4. Tibial component according to one of claims 1 to 3, **characterized in that** the anchoring keel (3) has a third wall section (8).

5. Tibial component according to claim 4, **characterized in that** the third wall section (8) is arranged between the first and second wall sections (4, 5) in a substantially bisecting manner in a plan view of the support plate (2).

6. Tibial component according to claim 4 or 5, **characterized in that** the third wall section (8), in a medial-lateral view, has a concavely shaped outer shape (9), a convex shaped outer shape (9) or a straight or bevelled outer shape (9) at least over a predetermined circumferential section.

7. Tibial component according to one of claims 5 or 6, **characterized in that** the transition region between the first wall section (4) and the third wall section (8) and between the second wall section (5) and the third wall section (8) is concave (10).

8. Tibial component according to any one of claims 1 to 7, **characterized in that** the thickness (D) of the wall sections (5, 6, 8) is between 1 and 8 mm, preferably between 2 and 4 mm.

9. Tibial component according to one of claims 1 to 8, **characterized in that** the first and/or the second wall section (5, 6) is arranged at least in sections at an angle (β) between 0° and 7° to the direction normal (N) to the support plate (2).

10. Tibial component according to one of claims 1 to 9, **characterized in that** the support plate (2) comprises a reception (11) for an inlay, wherein the inlay being connectable to the reception (11) in particular by a form closure, particularly preferably by a snap connection.

11. Tibial component according to one of claims 1 to 10, **characterized in that** the support plate (2) and the anchoring keel (3) are manufactured as separate components and connected to each other.

## Revendications

1. Composant tibial (1) d'une articulation artificielle du genou, ledit composant tibial comprenant une plaque d'appui (2) sur laquelle est disposée une quille d'ancrage (3) destinée à l'ancrage dans le tibia du porteur de l'articulation du genou, la quille d'ancrage (3) comportant une première portion de paroi (4) et une deuxième portion de paroi (5) qui sont reliées l'une à l'autre, les deux portions de paroi (4, 5) ayant, dans une vue antéropostérieure, une forme extérieure convexe (6, 7) au moins sur une portion circonférentielle définie,
**caractérisé en ce que**
les deux portions de paroi (4, 5) ont, dans une vue antéropostérieure, une forme sensiblement semi-circulaire ou semi-elliptique, la quille d'ancrage (3) étant disposée sur la plaque d'appui (2) de manière centrée, quant à la direction médio-latérale et quant à la direction antéropostérieure, par rapport au centre de la plaque d'appui (2).

2. Composant tibial selon la revendication 1, **caractérisé en ce que** les deux portions de paroi (4, 5) sont disposées, en vue de dessus de la plaque d'appui (2), de manière à former l'une par rapport à l'autre un angle (α) qui est compris entre 90° et 180°, de préférence entre 110° et 150°.

3. Composant tibial selon la revendication 1, **caractérisé en ce que** les deux portions de paroi (4, 5) ont, en vue de dessus de la plaque d'appui (2), la forme d'un arc, d'une partie d'ellipse, de parabole ou de polygone.

4. Composant tibial selon l'une des revendications 1 à 3, **caractérisé en ce que** la quille d'ancrage (3) comporte une troisième portion de paroi (8).

5. Composant tibial selon la revendication 4, **caractérisé en ce que** la troisième portion de paroi (8) est disposée, en vue de dessus de la plaque d'appui (2), sensiblement suivant la bissectrice entre les première et deuxième portions de paroi (4, 5).

6. Composant tibial selon la revendication 4 ou 5, **caractérisé en ce que** la troisième portion de paroi (8) a, en vue médio-latérale, au moins sur une portion circonférentielle définie, une forme extérieure concave (9), une forme extérieure convexe (9) ou une forme extérieure droite ou biseautée (9).

7. Composant tibial selon l'une des revendications 5 ou 6, **caractérisé en ce que** la zone de transition entre la première portion de paroi (4) et la troisième portion de paroi (8) et entre la deuxième portion de paroi (5) et la troisième portion de paroi (8) est concave (10).

8. Composant tibial selon l'une des revendications 1 à 7, **caractérisé en ce que** l'épaisseur (D) des portions de paroi (5, 6, 8) est comprise entre 1 et 8 mm, de préférence entre 2 et 4 mm.

9. Composant tibial selon l'une des revendications 1 à 8, **caractérisé en ce que** la première et/ou la deuxième portion de paroi (5, 6) sont disposées au moins par portions suivant un angle (β) compris entre 0° et 7° par rapport à la direction normale (N) à la plaque d'appui (2).

10. Composant tibial selon l'une des revendications 1 à 9, **caractérisé en ce que** la plaque d'appui (2) comprend un logement (11) destiné à un insert, l'insert pouvant être relié au logement (11) notamment par une liaison par complémentarité de formes, de manière particulièrement préférée par une liaison par encliquetage.

11. Composant tibial selon l'une des revendications 1 à 10, **caractérisé en ce que** la plaque d'appui (2) et la quille d'ancrage (3) sont réalisées sous forme de composants séparés et sont reliées l'une à l'autre.
